(19) **Europäisches Patentamt European Patent Office Office européen des brevets**

(11) **EP 4 624 923 A1**

(12) **EUROPEAN PATENT APPLICATION**
published in accordance with Art. 153(4) EPC

(43) Date of publication:
01.10.2025 Bulletin 2025/40

(21) Application number: 23909901.3

(22) Date of filing: 29.11.2023

(51) International Patent Classification (IPC):
*G01N 33/18* (2006.01)

(52) Cooperative Patent Classification (CPC):
G01N 33/18

(86) International application number:
PCT/CN2023/135108

(87) International publication number:
WO 2024/139971 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.12.2022 CN 202211688318

(71) Applicant: Jiyuan Qingyuan Water Treatment Co.,
Ltd.
Jiyuan, Henan 459019 (CN)

(72) Inventor: HE, Gaorong
Shanghai 200127 (CN)

(74) Representative: Loo, Chi Ching et al
Albright IP Limited
County House
Bayshill Road
Cheltenham, Gloucestershire GL50 3BA (GB)

(54) **MEASUREMENT METHOD AND SYSTEM FOR PRECIPITATION SPEED OF INORGANIC SALT IN WATER, ELECTRONIC DEVICE, AND STORAGE MEDIUM**

(57) Disclosed in the present invention are a measurement method and system for the precipitation speed of an inorganic salt in water, an electronic device, and a storage medium. The measurement method comprises the following steps : S1 : obtaining the type of an inorganic salt precipitated in water to be measured, one of inorganic carbon methyl orange alkalinity (Cmalk), a pH value, and total dissolved inorganic carbon (DIC) in said water, an element of a target inorganic salt contributing to non-inorganic carbon methyl orange alkalinity and an initial content of the element; S2 : continuously measuring a change value of a carbon dioxide concentration ($CO_2w$) in said water or a carbon dioxide concentration ($PCO_2$) in a gas phase in balance with said water over time in a closed constant-temperature and constant-pressure environment; S3 : obtaining the precipitation amount of the target inorganic salt according to data obtained in step S1 and step S2; and S4 : obtaining the precipitation speed of the target inorganic salt according to the precipitation amount obtained in step S3. Provided is a measurement method for the precipitation speed of an inorganic salt in water that achieves less manual intervention, a small measurement lower limit, and high precision.

S1 — Obtain the type of an inorganic salt precipitated in water to be measured, one of inorganic carbon methyl orange alkalinity (Cmalk), a pH value, and total dissolved inorganic carbon (DIC) in said water, a total dissolved solid (TDS) content, an element of a target inorganic salt contributing to non-inorganic carbon methyl orange alkalinity and the content of the element;

S2 — Continuously measure a change value of a carbon dioxide concentration (CO2w) in said water or a carbon dioxide concentration (PCO2) in a gas phase in balance with said water over time in a closed constant-temperature and constant-pressure environment;

S3 — Obtain the precipitation amount of the target inorganic salt according to data obtained in step S1 and step S2;

S4 — Obtain the precipitation speed of the target inorganic salt according to the precipitation amount obtained in step S3 and the time in S2

Fig 1

**Description**

**Technical Field**

The present invention relates to the technical field of water treatment, and more particularly, to a measurement method and system for the precipitation speed of an inorganic salt in water, an electronic device, and a storage medium.

**Background Art**

Scale is defined as the formation of an undesirably hard layer of salt on a surface; These surfaces may be the surfaces of heat exchangers, water pipes or vessels, pores of rocks, membranes, and membrane pores; Excessive scale will cause the decrease of heat exchange efficiency, the increase of delivery pressure and energy consumption, the decrease of oilfield production efficiency and the damage of oilfield related facilities, the decrease of reverse osmosis membrane efficiency and the damage of facilities; The main chemical methods used to control scale include adjusting the supersaturation of the inorganic salts forming scale, adding scale inhibitors to inhibit the precipitation of inorganic salts, etc; The most common mechanism for controlling scale is to control the crystallization reaction of these salts, i.e. to control the amount and precipitation speed of these salts from water. After judging the main scale types and calculating the supersaturation by combining the water quality parameters and application conditions (e.g. temperature) with the solubility product constant of inorganic salts and designing a scheme for adjusting the crystallization reaction characteristics of the target inorganic salts, the scheme needs to be verified in the laboratory and then put into practical application; One of the main contents in laboratory verification and practical application is to detect the amount and speed of target inorganic salt precipitation from water. In the following, the process of measuring the amount and rate of target inorganic salt precipitation from water in the laboratory is referred to as process 1, and the process of measuring the amount and speed of inorganic salt precipitation from water in field application is referred to as process 2.

The prior art in process 1 is a static scale inhibition method, such as GB/T16632-2008 Determination of scale inhibition performance of water treatment agents - Calcium carbonate precipitation method; HG/T2024-2009 Determination of scale inhibition performance of water treatment agents - Bubble method; HG/T4541-2013 Water treatment chemicals - Determination of scale inhibition performance - Limited carbonate method, the turbidity method disclosed in V. Tantayakom at all., Scale inhibition study by turbidity measurement, Journal of Colloid and Interface Science 284 (2005) 57-65 as well as the conductivity method, the critical pH method, the pH shift method, the dynamic simulation evaluation method, and the like. The above-mentioned methods are all used to characterize the amount of inorganic salt precipitated from water or the critical point at which precipitation starts to occur in a test solution with a certain degree of supersaturation, with some chemical or physical change of the test solution. The static scale inhibition and bubble method were used to calculate the scale inhibition rate (precipitation inhibition rate) with the change of the ion concentration of scale-forming inorganic salt; the limit carbonate method is used to continuously increase the supersaturation degree and periodically determine the concentration of relevant ions in the test. When it is measured that the ion concentration of scale-forming inorganic salts decreases, this is considered to be the critical point for inorganic salts to precipitate from water. For the calcium carbonate scale, the sum of total alkalinity and calcium ion concentration (both calculated as calcium carbonate) at this time is defined as the limit carbonate value; the conductivity method, critical pH method, and turbidity method were used to measure the breakthrough point of conductivity, pH and turbidity, respectively. The supersaturation at the breakthrough point was calculated, and the scale inhibition rate (precipitation inhibition rate), limit carbonate, critical supersaturation ratio, and critical supersaturation value were calculated based on this.

In the prior art in process 2, the ratio of ions such as potassium ions, sodium ions, and chloride ions which are not easily precipitated in water to the ions that may be precipitated also attempts to calculate the precipitation amount and speed of insoluble salts through the mass balance of the quantity of water and the concentration of ions in water; two kinds of fluorescent substances are added into the water in a certain proportion, one of which is synthesized on the scale inhibitor, and the other is considered not to be consumed in water. By monitoring the difference between the two kinds of fluorescent substances, the consumption of scale inhibitor is reflected, and it is considered that the precipitation of insoluble salt will consume the scale inhibitor, thus indirectly reflecting the precipitation amount and speed of insoluble salt, which is hereinafter referred to as 3D TRASAR™ method; monitor the heat exchanger and so on. Theoretically, by measuring the concentration change of the target inorganic ions in the water sample after constant temperature for a certain period of time, it is possible to calculate the precipitation speed of these inorganic substances from the water. Due to the low precipitation speed that must be controlled in a practical application environment and the limited precision and accuracy of prior art analysis of the concentration of these ions, this method cannot be used to achieve this goal.

The capillary method in the dynamic simulation test measures the degree of adsorption of inorganic salts on the capillary by measuring the pressure difference between two sections of the capillary under constant temperature and pressure. This method requires strict control of the capillary flow rate and therefore uses a constant flow pump with high accuracy but low tolerance to suspended matter in the water. Thus, the practical application in the field is limited.

## Summary of the Invention

It is an object of the present invention to provide a measurement method and a measurement system, an electronic device, and a storage medium for insoluble inorganic salts containing inorganic carbon methyl orange alkalinity which cause alkalinity change after precipitation with less human intervention, a small measurement lower limit, and a high precision of precipitation amount and precipitation speed.

In order to achieve the above-mentioned object, the technical solution adopted by the present invention is to provide a measurement method for the precipitation speed of an inorganic salt in water, comprising the following steps: S1: obtaining the type of an inorganic salt precipitated in water to be measured, one of inorganic carbon methyl orange alkalinity (Cmalk), a pH value, and total dissolved inorganic carbon (DIC) in the water, total dissolved solid (TDS) an element of a target inorganic salt contributing to non-inorganic carbon methyl orange alkalinity and a content of the element;

S2: continuously measuring a carbon dioxide concentration ($CO_2w$) in the water to be measured or a change value of a carbon dioxide concentration ($PCO_2$) in a gas phase in balance with it over time in a closed constant-temperature and constant-pressure environment; S3: obtaining the precipitation amount of the target inorganic salt according to data obtained in step S1 and step S2; and S4: obtaining the precipitation speed of the target inorganic salt according to the precipitation amount obtained in step S3 and the time in S2.

Preferably, the other two of the inorganic carbon methyl orange alkalinity (Cmalk), the $pH(t_0)$ value and the total dissolved inorganic carbon (DIC) in the water are calculated according to the following formulas in step S1, wherein

$$DIC = CO_2w \times (1 + k'_1/(H^+) + k'_1 \times k'_2/(H^+)^{\wedge}2), \quad\quad ----(1)$$

$$Cmalk = 50000 \times CO_2w \times (k'_1/(H^+) + 2k'_1 \times k'_2/(H^+)^{\wedge}2), \quad\quad ----(2)$$

$$pH = -\log(H^+) \quad\quad ----(3)$$

where DIC is the total dissolved inorganic carbon concentration in the water (mol/L), $CO_2w$ is the carbon dioxide concentration in the water to be measured (mol/L), Cmalk is the inorganic carbon methyl orange alkalinity in the water (mg/L, calculated as calcium carbonate), $H^+$ is a hydrogen ion concentration in the water to be measured (mol/L), $k'_1$ is a first dissociation constant of carbonic acid, and $k'_2$ is a second dissociation constant of carbonic acid.

Preferably, the target inorganic salt is calcium carbonate, and there are no other ions contributing to the inorganic carbon methyl orange alkalinity in the water to be measured, step S3 comprises the following steps:

S311: knowing $Cmalk(t_0)$ and $CO_2w(t_0)$ at $t_0$, and calculating $H^+(t_0)$ and $DIC(t_0)$ at the starting time $t_0$ according to formulas (2) and (1);

S312: knowing $CO_2w(t_1)$ at $t_1$, calculating $H^+(t_1)$ at $t_1$ according to formula (5), calculating $Cmalk(t_1)$ at $t_1$ according to formula (2), and calculating $DIC(t_1)$ at $t_1$ according to formula (4),

$$(DIC(t_0) - DIC(t_1)) \times 100 \times 1000 = (Cmalk(t_0) - Cmalk(t_1)) \quad\quad --(4)$$

$$2 \times CO_2w(t_0) - 2 \times CO_2w(t_1) = CO_2w(t_1) \times k'1/H^+(t_1) - CO_2(t_0) \times k'1/H^+(t_0) \quad --(5)$$

S313: calculating the calcium carbonate precipitation amount according to formula (6)

$$CaCO_3 (s)(mg/L) = (DIC(t_0) - DIC(t_1)) \times 10^{\wedge}5 \quad\quad --(6)$$

Preferably, the inorganic salt precipitated is calcium phosphate, and no ions are contributing to the inorganic carbon methyl orange alkalinity other than the inorganic carbon methyl orange alkalinity contributed by orthophosphate in the water to be measured, step S3 comprises the following steps: S321: knowing $Cmalk(t_0)$ and $CO_2w(t_0)$ at $t_0$, and calculating $H^+(t_0)$ and $DIC(t_0)$ at the starting time $t_0$ according to formulas (2) and (1);

S322: knowing $CO_2w(t_1)$ at $t_1$, calculating $DIC(t_1)$ and $H^+(t_1)$ at $t_1$ according to formula (7), calculating $Cmalk(t_1)$ at $t_1$ according to formula (2),

$$DIC(t_0) = DIC(t_1) = CO_2w(t_1) \times (1 + k'1/H^+(t_1) + k'1k'2/(H^+(t_1)^{\wedge}2) \quad --(7)$$

S323: knowing $Tp(t_0)$ at $t_0$, obtaining $DIP(t_0)$ according to formula (9), calculating $Pmalk(t_0)$ according to formulas (10)-(12), calculating $DIP(t_1)$ according to formulas (8)-(12), and calculating calcium phosphate precipitation amount according to formula (14);

$$(DIP(t_0)\text{-}DIP(t_1))/2 \times 2 \times 50 \times 1000 = Cmalk(t_0) + Pmalk(t_0) - Cmalk(t_1) - Pmalk(t_1) \text{ ---------(8)}$$

$$DIP = Tp/95/1000 \text{ -----------------------------------------------(9)}$$

$$Pmalk = (PO_4{}^{3-}) \times 2 \times 50000 + (HPO_4{}^{2-}) \times 1 \times 50000 \text{ -----------------(10)}$$

$$(PO_4{}^{3-}) = Tp/95/1000 \times kp1 \times kp2 \times kp3/((H+)\hat{}3 + (H+)\hat{}2 \times kp1 + (H+) \times kp1 \times kp2 + kp1 \times kp2 \times kp3) \tag{11}$$

$$(HPO_4{}^{2-}) = (PO_4{}^{3-}) \times (H^+)/kp3 \text{ --------------------------------------------(12)}$$

$$\text{Calcium phosphate precipitation amount } Ca_3(PO_4)_2(s) = (DIP(t_0\text{---})\text{-}DIP(t_1))/2 \times 310 \times 1000 \text{ --------(14)}$$

wherein Pmalk is the inorganic phosphorus methyl orange alkalinity (mg/L, calculated as calcium carbonate), DIP is the total dissolved inorganic phosphorus concentration (mol/L), and Tp is the total dissolved inorganic phosphorus concentration (mg/L, calculated as $PO_4{}^{3-}$); $kp_1$ is the first ionization constant of phosphoric acid, $kp_2$ is the second ionization constant of phosphoric acid, and $kp_3$ is the third ionization constant of phosphoric acid.

Preferably, the target inorganic salt is magnesium silicate, and no ions are contributing to the inorganic carbon methyl orange alkalinity other than the inorganic carbon methyl orange alkalinity contributed by silicate in the water to be measured, and step S3 comprises the following steps:

S331: knowing $Cmalk(t_0)$ and $CO_2(t_0)$ at $t_0$, and calculating $H^+(t_0)$ and $DIC(t_0)$ at the starting time $t_0$ according to formulas (2) and (1);
S332: knowing $CO_2(t_1)$ at $t_1$, calculating $DIC(t_1)$ and $H^+(t_1)$ at $t_1$ according to formula (7), calculating $Cmalk(t_1)$ at $t_1$ according to formula (2),

$$DIC(t_0) = DIC(t_1) = CO_2(t_1) \times (1 + k'1/H^+(t_1) + k'1k'2/(H^+(t_1)\hat{}2) \text{ --(7)}$$

S333: knowing $Tsi(t_0)$ at $t_0$, obtaining $DISi(t_0)$ according to formula (16), calculating $Simalk(t_0)$ according to formulas (17) and (18), and calculating $DISi(t_1)$ according to formulas (15)-(18), and calculating the magnesium silicate precipitation amount according to formula (19);

$$(DISi(t_0) - DISi(t_1)) \times 2 \times 50 \times 1000 = Cmalk(t0) + Simalk(t_0) - Cmalk(t_1) - Simalk(t_1) \text{ --(15)}$$

$$DISi = Tsi/60/1000 \text{ -- (16)}$$

$$Simalk = 50000 \times (Si(OH)_3O^-) \text{ --(17)}$$

$$(Si(OH)_3O^-) = DISi/(1 + H^+/Ksi) \text{ --(18)}$$

$$\text{magnesium silicate precipitation amount } MgSiO_3(s)(mg/L) = (DISi(t0)\text{-}DISi(t1)) \times 2 \times 50 \times 1000 \text{ -- (19)}$$

wherein DISi is the total dissolved inorganic silicon content (mol/L), Tsi is the total dissolved inorganic silicon content (mg/L, calculated as $SiO_2$), Simalk is the inorganic silicon methyl orange alkalinity (mg/L, calculated as calcium carbonate), and Ksi is the dissociation constant of silicic acid.

Preferably, the target inorganic salt is colloidal silicon, and no ions are contributing to the inorganic carbon methyl orange alkalinity other than the inorganic carbon methyl orange alkalinity contributed by silicate in the water to be measured, and step S3 comprises the following steps:

S341: knowing $Cmalk(t_0)$ and $CO_2(t_0)$ at $t_0$, and calculating $H^+(t_0)$ and $DIC(t_0)$ at the starting time $t_0$ according to formulas (2) and (1);

S342: knowing $CO_2(t_1)$ and $Cmalk(t_1)$ at $t_1$, calculating $DIC(t_1)$ and $H^+(t_1)$ at $t_1$ according to formula (7), calculating $Cmalk(t_1)$ at $t_1$ according to formula (2);

$$DIC(t_0) = DIC(t_1) = CO_2(t_1) \times (1 + k'1/H^+(t_1) + k''1 k'2/(H+(t_1)^\wedge 2)) \text{ --(7)}$$

S343: knowing $Tsi(t_0)$ at $t_0$, obtaining $DISi(t_0)$ according to formula (16), calculating $Simalk(t_0)$ according to formulas (17) and (18), and calculating $DISi(t_1)$ according to formulas (16)-(18) and (20), and calculating the colloidal silicon precipitation amount according to formula (21),

$$(DIsi(t_0) - DIsi(t_1)) \times 1 \times 50 \times 1000 = -(Cmalk(t_0) + Simalk(t_0)) + (Cmalk(t_1) + Simalk(t_1)) \text{ --(20)}$$

$$DISi = Tsi/60/1000 \qquad \text{--(16)}$$

$$Simalk = 50000 \times (Si(OH)_3 O-) = 50000 \times DISi/(1 + H^+/Ksi) \qquad \text{--(17)}$$

$$(Si(OH)_3 O^-) = DISi/(1 + H^+/Ksi) \qquad \text{--(18)}$$

colloidal silicon precipitation amount $SiO_2(s)$(mg/L)$= (DISi(t_0) - DISi(t_1)) \times 1 \times 60 \times 1000$ --(21)

Preferably, step S2 comprises: measuring a carbon dioxide gas concentration $PCO_2$ in a closed detector, wherein a gas circulation pump and a carbon dioxide concentration in a gas phase detector are provided in a loop of the closed detector, and continuously recording the carbon dioxide gas concentration $PCO_2$ during measurement; then converting the carbon dioxide gas concentration $PCO_2$ into the carbon dioxide concentration $CO_{2w}$ in water according to Henry's law, $CO_2w = Kh \times PCO_2$, and Kh is the Henry constant for carbon dioxide.

In order to achieve the above object, the present invention also provides an electronic device comprising: a processor; a memory, and a computer program stored in the memory and operable on the processor, when the computer program is executed by the processor, the above-mentioned measurement method for the precipitation speed of an inorganic salt in water is implemented.

In order to achieve the above object, the present invention also provides a computer-readable storage medium, a computer program is stored in the storage medium, and when the computer program is executed by a processor, the above-mentioned measurement method for the precipitation speed of an inorganic salt in water is implemented.

In order to achieve the above object, the present invention also provides a measurement system for the precipitation speed of an inorganic salt in water, comprising a closed detector for measuring the carbon dioxide concentration and the above-mentioned electronic device, the electronic device further comprising an input-output module, the output end of the closed detector being in signal communication with the input-output module of the electronic device.

Further, the closed detector comprises a test container, a carbon dioxide detector, a first constant-temperature heating unit, and a gas circulation pump, wherein water to be measured is placed in the test container, an inlet of the carbon dioxide detector is in communication with the test container, an outlet of the carbon dioxide detector is in communication with the test container via the gas circulation pump to form a pipeline loop for recirculating carbon dioxide, and the first constant-temperature heating unit is provided on the test container to control the temperature of the measured water in a constant temperature state.

The present invention has the following advantageous effects in comparison with the prior art: The present invention provides a measurement method and system for the precipitation speed of an inorganic salt in water, an electronic device, and a storage medium, wherein the measurement method is to calculate the precipitation speed of target inorganic salts in water by measuring the change over time of the carbon dioxide concentration in water or the carbon dioxide concentration in gas phase in balance with the concentration of carbon dioxide in the water under a closed constant-temperature and constant-pressure environment under the condition that the type of inorganic salt precipitated is confirmed in the previous water quality analysis and one of the inorganic carbon methyl orange alkalinity in water, pH, total dissolved inorganic carbon, the total dissolved solid content of water, and the inorganic carbon methyl orange alkalinity contributed by the target inorganic salts and the content thereof are known. The measurement method provided by the present invention can be automatically processed and calculated by a computer program, and the present invention can improve the efficiency of protocol evaluation when testing the precipitation speed of target inorganic salts in water in a laboratory and verifying the precipitation speed adjustment protocol, thus greatly improving the measurement accuracy and reducing the manual effort. Also, the present invention is applied to the on-site measurement of the precipitation speed of an inorganic salt in water and optimization of the precipitation speed adjustment scheme, providing a measurement system for the precipitation speed of an inorganic salt in water with a small measurement lower limit, less manual intervention, and a short measurement time, and providing an excellent tool for on-site automatic optimal adjustment of the precipitation speed of an inorganic salt. The present invention has great potential in water treatment intelligence, including, but not limited to, applications in the following fields: Circulating cooling water, surface water, geothermal water, drinking water, concentrated water in a reverse osmosis water system, concentrated water in thermal seawater desalination, water with deposition tendency of colloidal silicon dioxide and magnesium silicate, scaling measurement and regulation in brine vacuum salt production process, scaling measurement and regulation of related water systems in oil and gas exploitation, etc.

**Brief Description of the Drawings**

FIG. 1 is a schematic view showing a measurement flow for the precipitation speed of an inorganic salt in water according to an embodiment of the present invention;

FIG. 2 is a schematic view showing the structure of a carbon dioxide concentration detector according to an embodiment of the present invention;

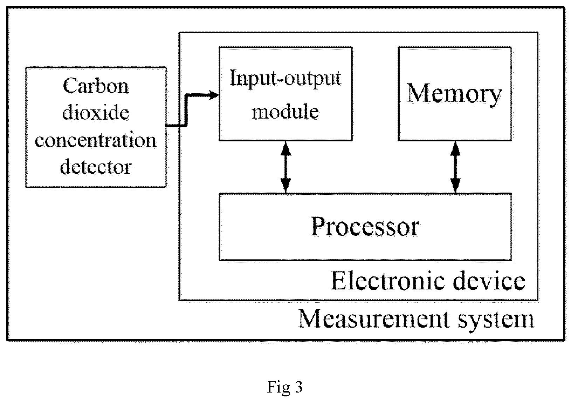

FIG. 3 is a block diagram of the principle of a measurement system for the precipitation speed of an inorganic salt in water according to an embodiment of the present invention;

FIGs. 4a and 4b are statistical plots of $PCO_2$ over time for the calcium carbonate precipitation rate measurements of the embodiments;

FIG. 5 is a schematic view showing a comparison between the calcium carbonate precipitation rates calculated by the inventive embodiments and the conventional alkalinity titration method;

FIG. 6a is a $PCO_2$ recording of field water and scale inhibitor supplemented field water at various test temperatures. FIG. 6b is the $PCO_2$ linear regression for the last 0.5 hours measured at 25°C, and FIG. 6c is the $PCO_2$ linear regression for the last 0.5 hours measured at 55°C and 75°C; and

FIG. 7 is a statistical plot of the results of calcium carbonate precipitation rate calculations for Type 1 field water.

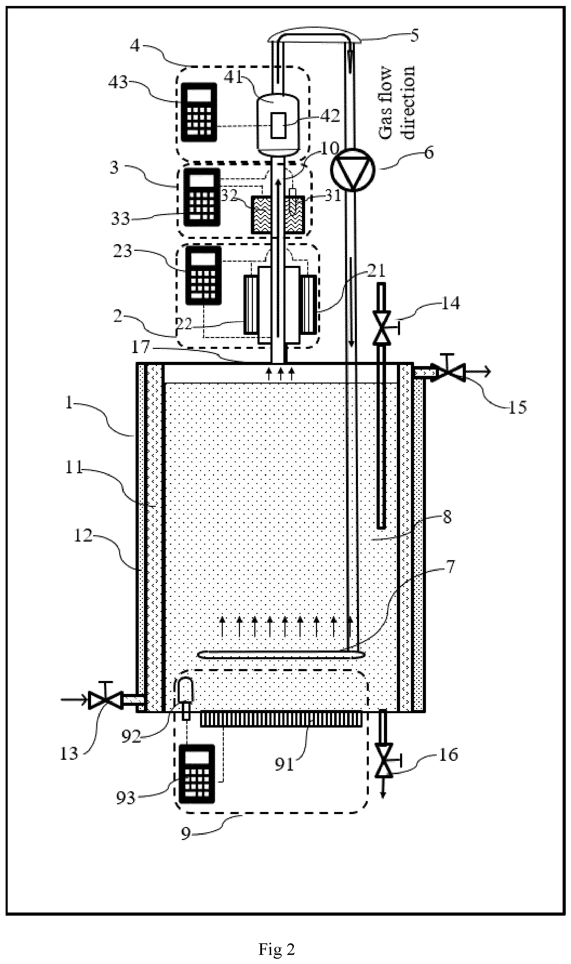

In FIG. 2:

1-test container, 10-pipeline, 11-jacket, 12-insulation layer, 13-jacketed water inlet valve, 14-test cup water inlet valve, 15-jacket water outlet valve, 16-test cup water outlet valve, 17-connection hole, 2-gas constant-temperature cooling unit, 21-first temperature sensor, 22-refrigeration sheet, 23-first controller, 3-second constant-temperature heating unit, 31-second temperature sensor, 32-heating wire, 33-second controller, 4-carbon dioxide detector, 41-measurement cavity, 42-carbon dioxide sensor, 43-display converter, 5-gas-phase pressure equalizing bag, 6-gas circulation pump, 7-gas distributor, 8-water to be measured, 9-first constant-temperature heating unit, 91-heater, 92-third temperature sensor, 93-third controller.

**Detailed Description of the Invention**

The invention will now be further described with reference to the accompanying drawings and examples.

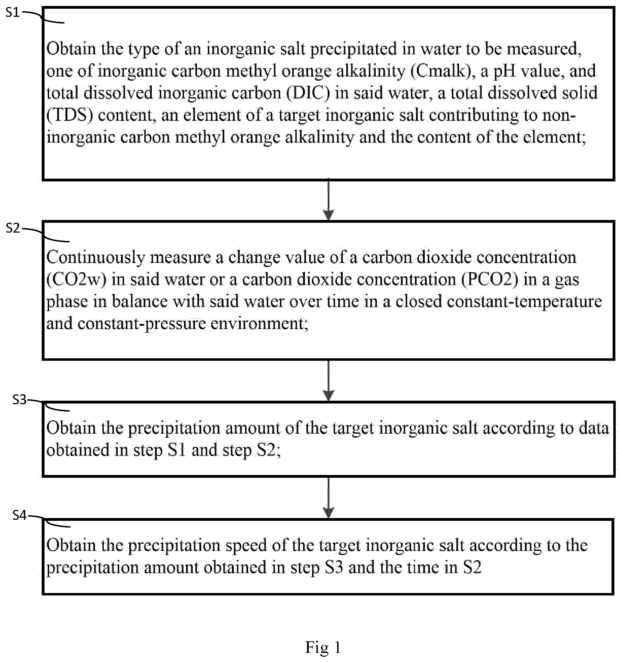

FIG. 1 is a schematic view showing a measurement flow for the precipitation speed of an inorganic salt in water according to an embodiment of the present invention.

Referring to FIG. 1, the measurement method for the precipitation speed of an inorganic salt in water provided in the present embodiment comprises the following steps:

S1: obtaining the type of an inorganic salt precipitated in water to be measured, one of inorganic carbon methyl orange alkalinity (Cmalk), a pH value, and total dissolved inorganic carbon (DIC) in said water, a total dissolved solid content of the test water (which can be converted from the electrical conductivity of the water), an element of a target inorganic salt contributing to non-inorganic carbon methyl orange alkalinity and an initial content of the element;

the alkalinity of water refers to the total amount of substances contained in the water that can quantitatively react with strong acids. The methyl orange alkalinity is the total amount of strong acid (120) contained in the water that can be quantitatively neutralized by the strong acid to the point at which the methyl orange indicator changes from yellow-orange to red-orange (solution pH4.4 to 4.5). Depending on the quality of the water, the source of the methyl orange alkalinity in the water varies.

Water containing inorganic carbon methyl orange alkalinity means water containing carbon dioxide, bicarbonate, and carbonate. The relationship between the three existing forms of inorganic carbon and the hydrogen ion concentration (H+) in water and the inorganic carbon methyl orange alkalinity (Cmalk) in water containing the inorganic carbon methyl orange alkalinity has been well studied and quantified. The following is a brief description:

$$DIC = CO_2w \times (1 + k'1/(H^+) + k'1 \times k'2/(H^+)^\wedge 2, \qquad ----(1)$$

$$Cmalk = 50000 \times CO_2w \times (k'1/(H^+) + 2 \times k'1 \times k'2/(H^+)^\wedge 2) \qquad ----(2)$$

$$pH = -\log(H^+) \qquad ----(3)$$

Where $CO_2w$ is the carbon dioxide concentration in the water to be measured, $H^+$ is the hydrogen ion concentration in the water to be measured, the negative logarithm thereof is the pH of the solution, k'1 is the first dissociation constant of carbonic acid corrected by the temperature and the ionic strength of water, k'2 is the second dissociation constant of carbonic acid corrected by the temperature and the ionic strength of water, and k'1 and k'2 can be selected or calculated from the available data and data according to the water quality characteristics of the water to be measured, the water temperature and the solid content of the water. It can be seen from the above-mentioned formulas (1), (2), and (3) that in the four of DIC, Cmalk, pH, and $CO_2w$, knowing any two of the four can know the other two; In a closed measurement system with constant temperature and pressure, if one of the initial DIC, Cmalk and pH of water is known, and then $CO_2w$ is measured, it is feasible to calculate the unknown two of the previous three.

Under certain conditions, the precipitation and dissolution of different kinds of inorganic salts in water occur. There are many existing knowledge and calculation software to judge and calculate the possible sediment types and thermodynamic tendencies in the target water. The present invention classifies into three categories whether the total methyl orange alkalinity (Tmalk) and the total dissolved inorganic carbon (DIC) in the water are altered after precipitation of the inorganic salts. Type 1 is both changed, type 2 is only changed Tmalk, and type 3 is both unchanged. Table 1 lists the classification results of the common inorganic scale according to this classification principle and the quantitative change relationship of the components in the water after precipitation. It can be seen from Table 1 that after calcium carbonate is precipitated, Cmalk and DIC of water are reduced, and the total dissolved inorganic phosphorus (DIP) and total dissolved inorganic silicon (DISi) are not changed; The precipitation of calcium sulfate did not change Tmalk and DIC, DIP, DISi; After calcium phosphate precipitation, pmalk and DIP of water decreased but DIC did not change; The precipitation of magnesium silicate reduces the Simalk of water but does not change the DIC; colloidal silicon is a self-polymer of silicic acid, and each molecule of silicic acid polymerizes to produce a hydroxide that increases the alkalinity of the water without altering the DIC. In summary, when type 1 or type 2 scale is produced in water, the Tmalk in the water will change, changing the balance of the carbonate system, causing a change in pH and $CO_2w$. According to the change value of $CO_2w$, combined with the quantitative relationship between the precipitation amount of type 1 scale and type 2 scale and Tmalk, DIC, DIP, and DIsi, the corresponding precipitation amount of inorganic salts can be calculated.

Table 1 Quantitative changes of components in water after precipitation of common insoluble inorganic salts

| Name | Chemical Formula | Molecular Weight | Change of Methyl Orange Alkalinity | | | | | Change of Element | | | Type |
|---|---|---|---|---|---|---|---|---|---|---|---|
| | | | Tmalk* | Cmalk | Pmalk | Simalk | OHmalk | DIC | DIP | DISi | |
| | | | (mg as $CaCO_3$/mmol Salt Precipitation) | | | | | mmol/mmol Salt Precipitation | | | |
| Calcium Carbonate | $CaCO_3$ | 100 | -100 | -100 | 0 | 0 | 0 | -1 | 0 | 0 | 1 |
| Calcium Sulfate | $CaSO_4$ | 136 | 0 | 0 | 0 | 0 | 0 | 0 | 0 | 0 | 3 |
| Calcium phosphate | $Ca_3(PO_4)_2$ | 310 | -200 | 0 | -200 | 0 | 0 | 0 | -2 | 0 | 2 |
| magnesium silicate | MgSiO3 | 100 | -100 | 0 | 0 | -100 | 0 | 0 | 0 | -1 | 2 |

(continued)

| Name | Chemical | Molecular | Change of Methyl Orange Alkalinity | | | | | Change of Element | | | Type |
|---|---|---|---|---|---|---|---|---|---|---|---|
| | Formula | Weight | Tmalk* | Cmalk | Pmalk | Simalk | OHmalk | DIC | DIP | DISi | |
| | | | (mg as CaCO$_3$/mmol Salt Precipitation) | | | | | mmol/mmol Salt Precipitation | | | |
| colloidal silicon | SiO$_2$ | 60 | 50 | 0 | 0 | 0 | 50 | 0 | 0 | -1 | 2 |
| *Tmalk = Cmalk + Pmalk + Simalk + OHmalk | | | | | | | | | | | |

S2: measuring a change value of a carbon dioxide concentration CO$_2$w in said water or a carbon dioxide concentration PCO$_2$ in a gas phase in balance with said water over time in a closed constant-temperature and constant-pressure environment;

For the measurement of carbon dioxide concentration, it is feasible to directly detect the concentration of CO$_2$w in the water or detect the concentration of PCO$_2$ in the gas phase space which is in balance with the water level, and then calculate the concentration of CO$_2$w of the carbon dioxide in the water according to the Henry constant Kh of carbon dioxide, i.e. calculated by PCO$_2 \times$ Kh.

Referring to FIG. 2, in one embodiment, a closed detector having a total volume of 500 ml with constant temperature heating and cooling is set up to detect the carbon dioxide concentration in a gas phase in balance with the water to be measured 8. A gas circulation pump 6 and a carbon dioxide detector 4 are provided in the closed detector loop. During the test, the carbon dioxide concentration in a gas phase was continuously recorded. Specifically, the closed detector comprises a test container 1, a carbon dioxide detector 4, a first constant-temperature heating unit 3, and a gas circulation pump 6, wherein water to be measured 8 is placed in the test container 1, an inlet of the carbon dioxide detector 4 is in communication with the test container 1, an outlet of the carbon dioxide detector 4 is in communication with the test container 1 via the gas circulation pump 6 to form a pipeline loop for recirculating carbon dioxide, and a first constant-temperature heating unit 9 is provided on the test container 1 to control the temperature of the test water in a constant-temperature state.

In a particular embodiment, the test container 1 can be a test cup with a diameter of 65 mm and a height of 150 mm coated with a Teflon coating inside, a test cup water inlet valve 14 and a test cup water outlet valve 16 are provided on the test cup, a jacket 11 is provided, the jacket 11 is wrapped outside the test container 1, a heat insulation layer 12 is provided on the outer side of the jacket 11, and a jacket water inlet valve 13 and a jacket water outlet valve 15 are provided on the jacket 11; A connection hole 17 is opened at the top cover of the test container 1, and the connection hole 17 is connected to a pipeline 10; in the present embodiment, the pipeline 10 is a 304 stainless steel pipe with an outer diameter of 16 mm and an inner diameter of 9 mm; a gas constant-temperature cooling unit 2 and a second constant-temperature heating unit 3 are successively provided outside the pipeline between the test container 1 and the carbon dioxide detector 4; the gas constant-temperature cooling unit 2 comprises a first temperature sensor 21, a refrigeration sheet 22 and a first controller 23, wherein the refrigeration sheet 22 is wrapped outside the pipeline, and a cooling surface of the refrigeration sheet 22 is tightly connected to an outer stainless steel pipe via metal tin; A first temperature sensor 21 is arranged between the cold sink 22 and the pipe, i.e. the first temperature sensor 21 is provided between the metallic tin and the stainless steel outer pipe, and a first controller 23 (PID control) is electrically connected to the first temperature sensor 21 and the cold sink 22, and controls the temperature in the metallic tin and the stainless steel pipe at a first predetermined value, which may be 5 ± 0.5°C, the stainless steel pipe length of the cooling section being 40 mm in the present embodiment. The second constant-temperature heating unit 3 comprises a heating wire 32, a second temperature sensor 31, and a second controller 33, wherein the heating wire 32 is wound outside a stainless steel pipe, and in the present embodiment, the heating wire 32 is formed by two Teflon low-voltage heating wires with an outer diameter of 1.2 mm and a resistance of 10 ohms per meter and wound in parallel for 20 turns, so as to form a heating section with a total length of 48 mm on the stainless steel pipe. A second temperature sensor 31 is disposed between the stainless steel outer pipe wall and the heating wire 32, and a second controller 33 (PID control) is electrically connected to the heating wire 32 and the second temperature sensor 31 to control the pipeline temperature at a second predetermined value, the second predetermined value being greater than the first predetermined value. In this embodiment, the second controller 33 controls the temperature of the stainless steel outer pipe wall to be 55 ± 0.5°C. The gas constant-temperature cooling unit 2 and the second constant-temperature heating unit 3 were set up to meet the requirements of the carbon dioxide sensor 41 (SenseAir S8/004-0-0053, manufactured by SenseAir, Sweden) selected in this test for measuring the humidity in the gas. By setting up the gas constant-temperature cooling unit 2 and the second constant-temperature heating unit 3, the measured gas is cooled first and then heated, so as to reduce the humidity in the measured gas. In other embodiments, the carbon dioxide sensor 41, the gas constant-temperature cooling unit 2, and the second constant-temperature heating unit 3, which are selected to have a high humidity adaptability, may be omitted.

After passing through the gas constant-temperature cooling unit 2 and the second constant-temperature heating unit 3 to reduce the humidity, the measurement gas enters a carbon dioxide detector 4, which comprises a measurement cavity 41, a carbon dioxide sensor 42, and a display converter 43. In the present embodiment, the measurement cavity 41 is a 316 stainless steel cylinder with an inner diameter of 25 mm and a height of 45 mm, the bottom of the measurement cavity 41 is connected to the upper part of the pipeline 10, and the top of the measurement cavity 41 is connected to the gas-phase pressure equalizing bag 5 via a 316 stainless steel capillary pipe with a diameter of 3 mm. A carbon dioxide sensor 42 is built in the measurement cavity 41, and the carbon dioxide sensor 42 is connected to a display converter 43, and the carbon dioxide concentration is measured and recorded once per minute by the display converter 43. The gas-phase pressure equalizing bag 5 is a Teflon film-coated aluminum foil gas bag having a length of 100 mm and a width of 80 mm, respectively. One of the two ports of the gas-phase pressure equalizing bag 5 is connected to the measurement cavity 41, and the other port is connected to a capillary pipe and then connected to the gas circulation pump 6, and the outlet of the gas circulation pump 6 is connected to the measurement container 1 via a 316 capillary pipe with a diameter of 3mm and passes the gas into the measurement water through the gas distributor 7, and the gas circulation flow rate is $60 \pm 20$ ml/min. The first constant-temperature heating unit 9 is used for heating and constant-temperature testing water and comprises a heater 91, a third temperature sensor 92 and a third controller 93, wherein the heater 91 is provided outside the bottom of the measurement container 1, the third temperature sensor 92 is provided inside the measurement container 1, and the third controller 93 is respectively electrically connected to the third temperature sensor 92 and the heater 91, and controls the temperature of the water to be measured 8 to be at a third pre-set temperature. When the test temperature is higher than 25 degrees centigrade, the test water is heated and thermostated using the first thermostatic unit 9. When the test temperature is equal to or less than 25°C, the test water temperature is maintained by the external constant-temperature water through the test cup jacket 11. According to the measurements, the volume of the whole test system was 500 ml at 25°C. The temperature of the test water was stabilized to the set point 20-25 minutes after the start of the test, as measured by the pre-measurement data of the system temperature control. Temperature deviation was at constant temperature $\pm 0.5$°C.

S3: obtaining the precipitation amount of the target inorganic salt according to data obtained in step S1 and step S2; and

In the first embodiment, the target inorganic salt is calcium carbonate, and there are no other ions contributing to the non-inorganic carbon methyl orange alkalinity in the water to be measured, which specifically comprises the following steps:

S311: knowing $Cmalk(t_0)$ and $CO_2w(t_0)$ at $t_0$, and calculating $H^+(t_0)$ and $DIC(t_0)$ at the starting time $t_0$ according to formulas (2) and (1);

S312: Having obtained $H^+(t_0)$ and $DIC(t_0)$ from S311, and knowing $CO_2w(t_1)$ at $t_1$, calculating $H^+(t_1)$ at $t_1$ according to formula (5), then calculating $DIC(t_1)$ according to formula (1), and calculating $Cmalk(t_1)$ according to formula (2),

The calculation procedure of formula (5) is as follows: Precipitation of the calcium carbonate reduces the total dissolved inorganic carbon in the water and the methyl orange alkalinity contained causes a reduction in the inorganic carbon methyl orange alkalinity in the water, both equal in value. On the left side of formula (4), the content of methyl orange alkalinity of precipitated calcium carbonate is shown, and on the right side of formula (4), the variation amount of inorganic carbon methyl orange alkalinity in water is shown, which are equal to each other. From formula (4), it can be calculated to formula (5)

$$(DIC_{(t0)}-DIC_{(t1)}) \times 100 \times 1000 = (Cmalk_{(t0)}-Cmalk_{(t1)}) \quad --(4)$$

∨

$$10^5 \times DIC_{(t0)} - 10^5 \times DIC_{(t1)} = Cmalk_{(t0)} - Cmalk_{(t1)}$$

∨

$$10^5 \times CO_2w(t_0) \times (1 + k'1/H^+_{(t0)} + k'1k'2/(H^+_{(t0)})^2 - 10^5 \times CO_2w(t_1) \times (1 + k'1/H^+_{(t1)} + k'1k'2/(H^+_{(t1)})^2 = CO_2(t_0) \times (50000 \times k'1/(H^+_{(t0)}) + 100000 \times k'1k'2/(H^+_{(t0)})^2) - CO_{2(t1)} \times (50000 \times k'1/(H^+_{(t1)}) + 100000 \times k'1k'2/(H^+_{(t1)})^2)$$

∨

$$2 \times CO_2w(t_0) - 2 \times CO_2w(t_1) = CO_2w(t_1) \times k'1/H^+(t_1) - CO_2w(t_0) \times k'1/H^+(t_0) \quad --(5)$$

S313: Calculate the calcium carbonate precipitation amount according to formula (6).

$$CaCO_3\ (s)(mg/L)= (DIC(t_0)-DIC(t_1)) * 10^5 \quad\quad --(6)$$

In a second embodiment, the target inorganic salt is calcium phosphate, and there is no ion contributing to the inorganic carbon methyl orange alkalinity other than the inorganic carbon methyl orange alkalinity contributed by orthophosphate in the water to be measured, which specifically comprises the following steps:

S321: knowing $Cmalk(t_0)$ and $CO_2w(t_0)$ at $t_0$, and calculating $H^+(t_0)$ and $DIC(t_0)$ at the starting time $t_0$ according to formulas (2) and (1);
S322: knowing $CO_2w(t_1)$ and $Cmalk(t_1)$ at $t_1$, calculating $DIC(t_1)$ and $H^+(t_1)$ at $t_1$ according to formula (7),

$$DIC(t_0) = DIC(t_1) = CO_2w(t_1)\times(1+k'1/H^+(t_1)+k'1k'2/(H^+(t_1)^2) \quad ----(7)$$

The non-carbonate precipitation does not change the DIC, and the $H^+(t_1)$ is obtained from formula (7).
S323: Knowing $Tp(t_0)$ at $t_0$, obtaining $DIP(t_0)$ according to formula (9), and calculating formula (13) according to formulas (10)-(12), then calculating $Pmalk(t_0)$ according to formula (13), calculating $DIP(t_1)$ and $Pmalk(t1)$ according to formulas (8) and (13), and calculating calcium phosphate precipitation amount according to formula (14);

$$(DIP(t_0)-DIP(t_1))/2\times2\times50\times1000 = Cmalk(t_0) + Pmalk(t_0)-Cmalk(t_1)-Pmalk(t_1)-(8)$$

$$DIP = Tp/95/1000------------------------------------------------(9)$$

$$Pmalk = (PO_4^{3-})\times2\times50000 + (HPO_4^{2-})\times1\times50000 --(10)$$

$$(PO_4^{3-}) = DIP \times kp1 \times kp2 \times kp3/((H + )^3 + (H + )2 \times kp1 + (H + ) \times kp1 \times kp2 + kp1 \times kp2 \times kp3) \quad\quad (11)$$

$$(HPO_4^{2-}) = (PO_4^{3-})\times(H^+)/kp_3 \quad\quad --(12)$$

$Pmalk = (DIP \times kp1 \times kp2 \times kp3/((H + )^3 + (H + )^2 \times kp1 + (H + ) \times kp1 \times kp2 + kp1 \times kp2 \times kp3)) \times 2 \times 50000 + (DIP \times kp1 \times kp2 \times kp3/((H + )^3 + (H + )^2 \times kp1 + (H + ) \times kp1 \times kp2 + kp1 \times kp2 \times kp3)) \times (H + )/kp3 \times 1 \times 50000$

$$Calcium\ phosphate\ precipitation\ amount\ Ca_3(PO_4)_2(s)=(DIP(t_0---)-DIP(t_1))/2\times310\times1000 --(14)$$

wherein Pmalk is the inorganic phosphorus methyl orange alkalinity (mg/L, calculated as calcium carbonate), DIP is the total dissolved inorganic phosphorus (mol/L), and Tp is the total dissolved inorganic phosphorus content (mg/L, calculated as $PO_4^{3-}$); kp1 is the first ionization constant of phosphoric acid, kp2 is the second ionization constant of phosphoric acid, and kp3 is the third ionization constant of phosphoric acid.
After calcium phosphate precipitation, the total dissolved inorganic phosphorus (DIP) in the water and the methyl orange alkalinity (non-inorganic carbon methyl orange alkalinity) contributed by phosphate are decreased. On the left side of formula (8), the methyl orange alkalinity expressed by the precipitated calcium phosphate is decreased, and on the right side, the methyl orange alkalinity in the water is decreased, both of which should be equal.
$CO_2w(t_0) = PCO_2t_0 \times Kh$ and $Cmalk(t_0)$ is a known value, $H^+(t_0)$ can be calculated according to formula (2), and then $DIC(t_0)$ can be obtained according to formula (1). Calcium phosphate does not contain carbonate and does not change DIC in water after precipitation from water, i.e. $DIC(t_1) = DIC(t_0)$ Thus, $H^+(t_1)$ can be obtained according to formula (7), and $Cmalk(t_1)$ can be obtained according to formula (2). So far, only $DIP(t_1)$ and $Pmalk(t_1)$ in formula (8) are unknown, and $DIP(t_1)$ and $Pmalk(t_1)$ need to satisfy formula (13); therefore, $DIP(t_1)$ and $Pmalk(t_1)$ can be solved by formula (8) and formula (13). One method for solving this problem is the trial difference method of $DIP(t_1)$. By setting different values of $DIP(t_1)$, $DIP(t_1)$ and $Pmalk(t_1)$ satisfying formula (8) and formula (13) are calculated, and then the precipitation amount of calcium phosphate is obtained according to formula (14).
In a third embodiment, the target inorganic salt is magnesium silicate, and the water to be measured has no ions contributing to the inorganic carbon methyl orange alkalinity other than the inorganic carbon methyl orange alkalinity contributed by the silicate, which specifically comprises the following steps:

S331: knowing $Cmalk(t_0)$ and $CO_2w(t_0)$ at $t_0$, and calculating $H^+(t_0)$ and $DIC(t_0)$ at the starting time $t_0$ according to formulas (2) and (1);

S332: knowing $CO_2w(t_1)$ and $Cmalk(t_1)$ at $t_1$, calculating $DIC(t_1)$ and $H^+(t_1)$ at $t_1$ according to formula (7);

$$DIC(t_0) = DIC(t_1) = CO_2w(t_1) \times (1 + k'1/H^+(t_1) + k'1k'2/(H^+(t_1)^\wedge 2))----(7)$$

S333: knowing $Tsi(t_0)$ at $t_0$, obtaining $DISi(t_0)$ according to formula (16), calculating $Simalk(t_0)$ according to formulas (17) and (18), and calculating $DISi(t_1)$ according to formulas (15)-(18), and calculating the magnesium silicate precipitation amount according to formula (19);

$$(DISi(t_0) - DISi(t_1)) \times 2 \times 50 \times 1000 = Cmalk(t_0) + Simalk(t_0) - Cmalk(t_1) - Simalk(t_1) \; --(15)$$

$$DISi = Tsi/60/1000 \qquad -- (16)$$

$$\text{magnesium silicate precipitation amount } MgSiO_3(s)(mg/L) = (DISi(t0)-DISi(t1)) \; \times 2 \times 50 \times 1000 \; -- (19)$$

wherein DISi is the total dissolved inorganic silicon (mol/L), Tsi is the total dissolved silicon content (mg/L, calculated as $SiO_2$), Simalk is the inorganic silicon methyl orange alkalinity (mg/L, calculated as calcium carbonate), and Ksi is the dissociation constant of silicic acid.

The alkalinity in the water decreases after the magnesium silicate is precipitated. On the left side of formula (15), the methyl orange alkalinity expressed by the precipitated magnesium silicate is shown, and on the right side, the alkalinity shown in the water decreases, and both of them need to be equal.

$CMalk(t_0)$ and $CO_2w(t_0)=PCO_2(t_0)*Kh$ are known values, and $H^+(t_0)$ can be calculated according to formula (2), and then $DIC(t_0)$ can be obtained according to formula (1). Magnesium silicate precipitates from water, reducing the methyl orange alkalinity of water and causing a decrease in pH. Since the non-carbonate basicity does not contain inorganic carbon, the DIC of the water remains unchanged. According to formula (7), $H^+(t_1)$ and then $Cmalk(t_1)$ can be obtained. It can be seen from the molecular formula of magnesium silicate that when 1mol/L of magnesium silicate is precipitated while reducing 1mol/L of total dissolved silicon, the alkalinity calculated as calcium carbonate of $2 \times 50 \times 1000$ mg/L will be reduced, namely, $(DISi(t_0)-DISi(t_1)) \times 2 \times 50 \times 1000=Talk(t_0)-Talk(t_1)$. Knowing the total dissolved inorganic silicon concentration at $t_0$, $Tsi(t_0)$, and knowing the $H^+(t_0)$ at $t_0$, the basicity contributed by the silicon at $t_0$, $Simalk(t_0)$, can be calculated from the dissociation constant of silicic acid. When a value of $DISi(t_1)$ is set, $Simalk(t_1)$ can be calculated from the dissociation constant constant of $H^+(t_1)$ and silicic acid. Therefore, trial and error calculation can be performed on $DISi(t_1)$ to find $DISi(t_1)$ satisfying formula (15), and then the precipitation amount of magnesium silicate can be obtained according to formula (19).

In a fourth embodiment, the target inorganic salt is colloidal silicon, and no ions are contributing to the inorganic carbon methyl orange alkalinity other than the inorganic carbon methyl orange alkalinity contributed by silicate in the water to be measured, and the specifically comprises the following steps:

S341: knowing $Cmalk(t_0)$ and $CO_2w(t_0)$ at $t_0$, and calculating $H^+(t_0)$ and $DIC(t_0)$ at the starting time $t_0$ according to formulas (2) and (1);

S342: knowing $CO_2w(t_1)$ and $Cmalk(t_1)$ at $t_1$, calculating $DIC(t_1)$ and $H^+(t_1)$ at $t_1$ according to formula (7), calculating $Cmalk(t_1)$ at $t_1$ according to formula (2);

$$DIC(t_0) = DIC(t_1) = CO_2(t_1) \times (1+k'1/H^+(t_1)+k'^1k'2/(H+(t_1)^\wedge 2)) \; --(7)$$

S343: knowing $Tsi(t_0)$ at $t_0$, obtaining $DISi(t_0)$ according to formula (16), calculating $Simalk(t_0)$ according to formulas (17) and (18), and calculating $DISi(t_1)$ according to formulas (16)-(18) and (20), and calculating the colloidal silicon precipitation amount according to formula (21);

$$(DIsi(t_0)-DIsi(t_1)) \times 1 \times 50 \times 1000 = -(Cmalk(t_0) + Simalk(t_0)) + (Cmalk(t_1) + Simalk(t_1))-(20)$$

$$DISi = Tsi/60/1000 \qquad --(16)$$

$$Simalk = 50000 \times (Si(OH)_3O^-) = 50000 \times DISi/(1 + H^+/Ksi) \qquad --(17)$$

$$\text{colloidal silicon precipitation amount } SiO_2(s)(mg/L) = (DISi(t_0) - DISi(t_1)) \times 1 \times 60 \times 1000 \; --(21)$$

$Cmalk(t_0)$ and $CO_2w(t_0) = PCO_2(t_0)*Kh$ are known values, and $H^+(t_0)$ can be calculated according to formula (2), and then $DIC(t_0)$ can be calculated according to formula (1); since colloidal silicon does not contain inorganic carbon, the DIC of water remains unchanged. According to formula (7), $H^+(t_1)$ and then $Cmalk(t_1)$ can be obtained.

Colloidal silicon is the self-polymerization of orthosilicic acid, and each orthosilicic acid, after polymerization, produces a hydroxide in water and increases in alkalinity in water. On the left side of the formula (20) is a quantitative expression that the precipitated colloidal silicon increases the alkalinity of water while decreasing the total dissolved inorganic silicon (DISi, mol/L), and on the right side is the alkalinity increase exhibited in water, both of which need to be equal.

Knowing the total dissolved inorganic silicon concentration Tsi at $t_0$, $DISi(t_0)$ can be obtained from formula 16, and then the basicity $Simalk(t_0)$ contributed by silicon at $t_0$ can be calculated according to the known dissociation constant of $H^+(t_0)$ combined with dissociation constant of silicic acid at $t_0$. Thus, in formula (20), $DISi(t_1)$ and $Simalk(t_1)$ remain unknown, $DISi(t_1)$ and $Simalk(t_1)$ satisfy formula (17) again, and $H^+(t_1)$ is a known value. Therefore, $DISi(t_1)$ and $Simalk(t_1)$ can be solved according to formula (20) and formula (17). One method of solving this problem is the $DISi(t_1)$ trial-and-error method, finding the $DISi(t_1)$ satisfying formulas (20) and (17), and then obtaining the precipitation amount of colloidal silicon according to formula (21).

S4: The precipitation speed of the target inorganic salt is obtained based on the precipitation amount obtained in step S3.

The precipitation amount is divided by the time $(t_1-t_0)$ at which the precipitation amount occurs, i.e. the average precipitation speed over the test period is obtained.

The present embodiment also provides an electronic device, comprising: a processor; a memory, and a computer program stored in the memory and operable on the processor, when the computer program is executed by the processor, the above-mentioned measurement method for the precipitation speed of an inorganic salt is implemented.

The present embodiment also provides a computer-readable storage medium storing a computer program that, when being executed by a processor, the above-mentioned measurement method for the precipitation speed of an inorganic salt is implemented.

**Example 1**

This example calculates the calcium carbonate precipitation speed from the results of the carbon dioxide concentration $PCO_2$ in gas phase measurement according to calculation software developed in the present invention.

It is known that the water to be measured has a tendency to precipitate calcium carbonate, and there are no other ions contributing to the inorganic carbon methyl orange alkalinity in the water to be measured. This water was known to have a Cmalk of 320 mg/L and a TDS of 1118 mg/L. 470 ml of test water was added into the test device, the test water temperature was set as 25°C, the test device was started, and $PCO_2$ was continuously measured and recorded. After one hour, the test was ended. From the $PCO_2$ recorded in the test, $PCO_2 = 994$ ppmv at 30 minutes and $PCO_2 = 1099$ ppmv at 60 minutes of the test. Table 2 lists the specific steps for calculating the calcium carbonate precipitation amount by calculation software developed in accordance with the present invention. The precipitation amount calculated from Table 2, divided by the time that the precipitation amount occurred, gave an average precipitation rate over the test period of time of 1.8/0.5 = 3.6 mg/L/hour.

Table 2 Calculation process of calcium carbonate precipitation amount

| | |
|---|---|
| Test water temperature (T), °C | 25 |
| Volume of test water (Vw), ml | 470 |

(continued)

| | Correlation constant value formula | Calculated Value |
|---|---|---|
| Total dissolved solids (TDS), mg/L | 1118 | |
| K'w, the dissociation constant of water at set temperature and ionic strength | 10^(6.0486-4471.33/(T + 273.15)-0.017053*(T + 273.15)-2×logfm | 1.28E-14 |
| k1', first order dissociation constant for carbonic acid at set temperature and ionic strength | 10^(14.8435-3404.71/(T + 273.15)-0.032786*(T + 273.15)-2×logfm | 6.16E-07 |
| k2', second order dissociation constant of carbonic acid at set temperature and ionic strength | 10^(6.498-2909.39/(T + 273.15)-0.02379*(T + 273.15)-logfd | 8.49E-11 |
| Ks', calcium carbonate solubility product constant at set temperature and ionic strength | 10^(13.87-3059/(T + 273.15)-0.04035*(T + 273.15)-2×logfd | 1.39E-08 |
| log Kh | = 108.3865 + 0.01985076*(T + 273.15)-6919.53/(T + 273.15)-40.45154*LOG(-T + 273.15)+ 669365/(T + 273.15)^2 | -1.47E + 00 |
| Kh $CO_2$, Henry constant for carbon dioxide at set temperature, (mol/L)/ppmv | = 10^logKh*10^-6 | 3.405E-0 8 |
| Logfm, the logarithm of the first order activity constant fm | A x ((I^0.5/(1 + I^0.5)-0.2*I)) | -0.07050 468 |

(continued)

|  | | Correlation constant value formula | | Calculate d Value | |
|---|---|---|---|---|---|
| Logfd, the logarithm of the second-order activity constant fd | | 4*logfm | | -0.28201 8719 | |
| A | | 1.825*10^6*(E*(T + 273.15))^-1.5 | | 0.512217 497 | |
| E, the dielectric constant of water | | 60954/(T + 389.15)-68.937 | | 78.24155 849 | |
| I. Ionic Intensity | | 2.5*10^-5*TDS | | 0.02795 | |
| I/O and computational logic interpretation | | | | | |
| Type of scale | | Type 1, precipitate calculated as calcium carbonate | | Input value and the calculatio n result | |
| Variation law of ions in water after scale precipitation | | $(DIC_{t0}-DIC_{t1}) \times 100000 = Cm\ alk_{to}-Cmalk_{t1}$ | | | |
| | | Time, $t_0$ | Time, $t_1$ | Ti me , $t_0$ | Ti me , $t_1$ |
| carbon dioxide concentration in gas phase Concentration ($PCO_2$), ppmv | | The measured values are entered into the Input and Calculation Results | | **99 4** | **10 99** |
| Carbon dioxide concentration in water $CO_2$w, mol/L | | Be calculated by $PCO_2$ x Kh | | 3.3 8E -0 5 | 3.7 4E -0 5 |
| Inorganic carbon CMalk, mg/L calculated as $CaCO_3$ | The basicity of inorganic carbon at to shall be input | | | **32** **0.0** | 31 8.2 |
| Total dissolved inorganic carbon (DIC), mol/L | From formulas (2) and (3), the pH at to can be found. One way to solve this problem is the trial-and-error method, namely, setting different pH values, and finally obtaining the pH value at which $Cmalk(t_0)$ is equal to the given value. After the pH is obtained, $DIC_{t0}$ can be obtained by formula (2) | Calculating $H^+(t_1)$ at $t_1$ according to formula (5), and using a pH trial and error method, obtaining $Cmalk(t_1)$ according to formula (2), calculating $DIC(t_1)$ at $t_1$ according to formula (4), and calculating $pH(t_1)$ at $t_1$ according to formula (3), | | 6.2 8E -0 3 | 6.2 6E -0 3 |
| pH | | | | 8.4 7 | 8.4 2 |
| Need to maintain an equal amount between t0 and t1 | $DIC(t_0)*10^5-Cmalk(t_0)$ | | $10^5 \times CO_2w_{t1} \times (1 + k'1/(H^+_{t1}) + k'1k'2/(H^+_{t1})^2)-CO_2W_{t1} \times (50000*k'1/H^+_{t1} + 100000 \times k'1k'2/(H^+_{t1})^2)$ | 3.0 8E + 02 | 3.0 8E + 02 |

(continued)

| | Correlation constant value formula | Calculate d Value |
|---|---|---|
| **Calculated precipitation amount in Calcium Carbonate, CaCO$_3$ (s), mg/L** | | **1.8** |

The specific application of the present invention in laboratory testing of the crystallization reaction characteristics of the target inorganic salt (Process 1) is further described below.

**Example 2**

This example is a calcium carbonate crystallization precipitation test.

In the laboratory, in order to evaluate the crystallization reaction characteristics of the target inorganic salt precipitated from water, one of the existing methods was the static scale inhibition method. Two kinds of stock solutions were prepared. When the two kinds of stock solutions were mixed and under a certain test environment, the crystallization of the target inorganic salt would be produced. By analyzing the concentrations of the target ions before and after the test, the precipitation characteristics of the target inorganic salts were quantitatively evaluated. In this test, while obtaining the calcium carbonate precipitation rate using the carbon dioxide concentration measurement device and calculation method of the present invention, the total methyl orange alkalinity of the water before and after the test was analyzed to obtain a set of data similar to that of the conventional static scale inhibition test, thereby calculating the calcium carbonate precipitation amount and deposition rate from the data and comparing the two sets of data.

1. Preparation for test

1) Preparation of stock solution of 0.5 mg (effective) ATMP/ml

0.25 g of 50% ATMP (a commonly used scale inhibitor, aminotrimethylene phosphinic acid, purchased from Taobao, Yousuo sample Xiaolindian) was weighed into a 200 ml beaker, about 100ml deionized water was added, mixed well, pH was adjusted to $7.0 \pm 0.1$ with 0.1 N NaOH, and then deionized water was used to make constant volume to 250ml.

2) Preparation of stock solutions A and B

Stock solution A: 1.813 g of NaHCO$_3$ (analytically pure, traditional Chinese medicine) was weighed into a 200 ml beaker, dissolved with about 100 ml of deionized water, and transferred to a 2 L volumetric flask, 0.127 g of Na$_2$CO$_3$ (analytically pure, traditional Chinese medicine) was weighed into a 200ml beaker, dissolved with 100 ml of deionized water and transferred to a 2 L volumetric flask, the 200 ml beaker was washed with deionized water for 2-3 times, and the wash solution was transferred to a 2 L volumetric flask. The stock solution A was repeatedly prepared once, and the twice prepared stock solution A was put into a 5-liter glass bottle after mixing, and then the bottle cap was tightly covered for future use.

Stock solution B: 1.763 g of CaCl$_2$ * 2H$_2$O (analytically pure, Fuchen (Tianjin) Chemical Reagent Co. Ltd.) was weighed into a 200 ml beaker, dissolved with about 100 ml of deionized water, transferred to a 2 L volumetric flask, then the 200 ml beaker was washed with deionized water for 2-3 times, the wash solution was transferred to the 2 L volumetric flask, deionized water was used to make constant volume to the 2 L volumetric flask. The stock solution B was repeatedly prepared, and the twice prepared stock solution B was put into a 5-liter glass bottle after mixing, and then the bottle cap was tightly covered for future use.

3) Titration of initial methyl orange alkalinity: a volumetric flask was used to take 250 ml of stock solution A, another volumetric flask was used to take 250ml of deionized water, and the two liquids were mixed and stored in a 500 ml glass bottle with cover. The sample was filtered with a 0.45-micron filter membrane (water system MCE 25 mm, filtration technology), the sample was transferred with a 50 ml pipette into a 200 ml conical flask, 3-5 drops of 0.1% methyl orange indicator was added, and then titrated with 0.05N HCl standard solution (Taobao, laboratory standard reagent business). Each sample was repeated 3 times, averaged, and the standard deviation was calculated.

2. Calcium Carbonate Crystallization Test

A 250 ml volumetric flask was used to take 250 ml of the stock solution A and was put into a 1000 ml beaker; according to the test design, different amounts of ATMP stock solution were added; another 250 ml volumetric flask was used to take 250 ml of the stock solution B and was put into a 1000 ml beaker in which the stock solution A had been stored, and then a glass rod was used to stir until uniform. The calculated water quality of the two stock solutions after mixing in equal amounts is shown in Table 3. According to the relevant calculation software, it could be seen that the calcite supersaturation index of the test solution was 1.68 at 25°C. 470 g of the test solution was taken, and placed in the test device, the test constant temperature was set at 25°C, and the test time was 1 h. $PCO_2$ was continuously recorded during this period and plotted over time after the end of the test. After 1 hour, the test solution was filtered with a 0.45-micron filter membrane, the sample was transferred with a 50 ml pipette into a 200 ml conical flask, 3-4 drops of methyl orange indicator was added, and then 0.05N HCl was used to titrate. The sample was repeated 3 times, averaged, and calculated the standard deviation.

Table 3. Water quality of calcium carbonate precipitation test

| | |
|---|---|
| $Na^+$, mg/L | 138 |
| $Ca^{2+}$, mg/L as $CaCO_3$ | 300 |
| Cmalk, mg/L as $CaCO_3$ | 300 |
| $Cl^-$, mg/L | 212 |
| PH, room temperature | 8.63 |
| Supersaturation Index SI at 25°C calculated for calcite (chemical composition calcium carbonate) from aqion* | 1.68 |
| *aqion is a free water quality calculation software that can determine the dissolution and deposition tendencies of 261 minerals based on water quality. Download web site: https: //www.aqion.de/direct/8.1.2_EN | |

3. Test Results

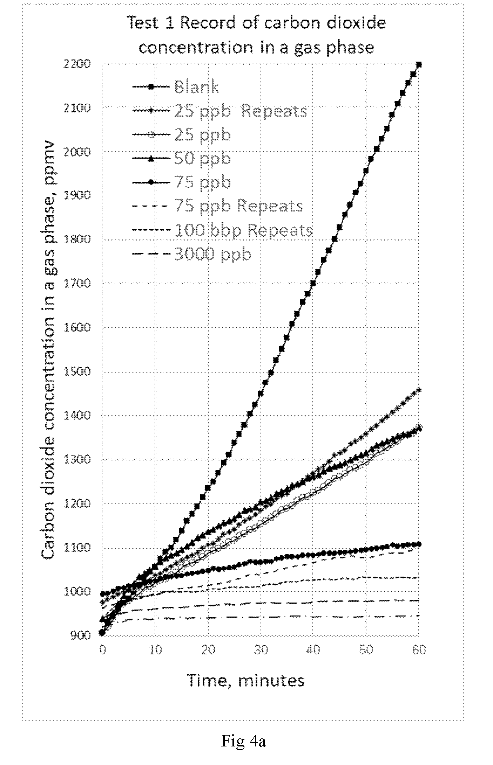

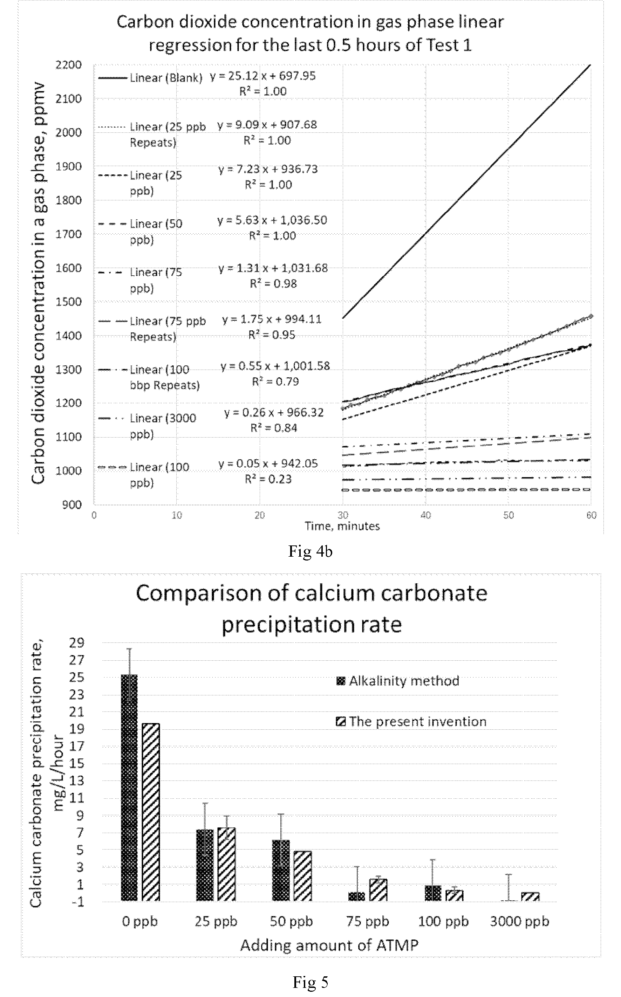

Table 4 shows the test arrangement and the statistics of test results. FIG. 4a and FIG. 4b are statistical plots of PCO2 over time.

It can be seen from the measurement of the constant temperature characteristics of the previous test device that the water temperature can be kept constant at 25 ± 0.5°C only after about 20-25 minutes from the start of the test. Therefore, the slope and intercept were calculated by linear regression of the data from the test time of 30 minutes to 60 minutes, see FIG. 5, and the $PCO_2$ of 0 minutes and 60 minutes were calculated from the slope and intercept, and then the calcium carbonate precipitation amount and the deposition rate were calculated from the values. The results of the final calculations are collectively listed in Table 4.

Table 4. Calcium Carbonate Crystallization Test Arrangement and Results

| Test No. | | 1# | 2# | 3# | 4# | 5# | 6# | 7# | 8# | 9# |
|---|---|---|---|---|---|---|---|---|---|---|
| Test Abbreviation | | 25 ppb | Bla nk | 100 ppb | 50 ppb | 75 ppb | 300 0 ppb | 75 ppb repeat s | 100 bbp repeat s | 25 ppb repeat s |
| Adding amount of ATMP, $\mu$g/L based on the active substance | | 25 | 0 | 100 | 50 | 75 | 300 0 | 75 | 100 | 25 |
| 500 ml volume of 0.5 mg (active) ATMP/ml to be added to the test solution, $\mu$l | | 25 | 0 | 100 | 50 | 75 | 300 0 | 75 | 100 | 25 |
| Initial Inorganic Carbon Alkalinity, mg/L as $CaCO_3$ | Theoretical formulation concentration | 300 | | | | | | | | |
| | Test 1 | 322.2 | | | | | | | | |
| | Test 2 | 320.8 | | | | | | | | |
| | Test 3 | 318.5 | | | | | | | | |
| | Avg | **320.5** | | | | | | | | |
| | Standard Deviation | 1.9 | | | | | | | | |
| Initial $Ca^{2+}$ (calculated according to theoretical preparation value), mg/L is calculated as $CaCO_3$ | | **300** | | | | | | | | |
| Conductivity, $\mu$s/cm (room temperature) | Measured value | 119 9 | 124 5 | 122 8 | 125 1 | Not Test ed | 122 3 | 1258 | 1258 | 124 |
| | Avg | **1240** | | | | | | | | |
| | Standard Deviation | 21 | | | | | | | | |
| Total dissolved solids*, mg/L | | **868** | | | | | | | | |
| Pre-test pH | Measured@Te mp°C | 8.67 @1 7.3 | 8.60 @1 5.8 | 8.62 @1 5.8 | 8.62 @1 8.2 | Not Test ed | 852 @1 6.2 | 8.67 @16. 9 | 8.66 @17. 2 | 8.70 @16. 7 |
| | Avg | **8.63** | | | | | | | | |
| | Standard Deviation | 0.06 | | | | | | | | |
| Supersaturation Index SI at 25°C calculated for calcite | | **1.68** | | | | | | | | |
| (chemical constituent calcium carbonate) from aqion* | | | | | | | | | | |
| Test temperature, °C | | **25** | | | | | | | | |

17

| Test No. | | 1# | 2# | 3# | 4# | 5# | 6# | 7# | 8# | 9# |
|---|---|---|---|---|---|---|---|---|---|---|
| Alkalinity measured after the test, mg/L is calculated as CaCO₃ | Test 1 | 312. 8 | 290. 2 | 320. 7 | 315. 7 | 319. 8 | 318. 3 | 320.1 | 321.3 | NA |
| | Test 2 | 310. 3 | 300. 2 | 322. 4 | 314. 7 | 323. 9 | 320. 3 | 322.8 | 319.8 | NA |
| | Test 3 | 316. 2 | 295. 2 | 321. 4 | 312. 7 | 317. 3 | 325. 3 | 318.5 | 318.3 | NA |
| | Avg | **313. 1** | **295. 2** | **321. 5** | **314. 4** | **320. 3** | **321. 3** | **320.5** | **319.8** | **NA** |
| | STDEV | **3.0** | **5.0** | **0.9** | **1.5** | **3.3** | **3.6** | **2.2** | **1.5** | **NA** |
| Calcium carbonate precipitation amount calculated from alkalinity, mg/L calculated as CaCO₃ | | **7.4** | **25.3** | **-1.0** | **6.1** | **0.2** | **-0.8** | **0.0** | **0.7** | **NA** |
| Slope, ppmv/hour, 0.5 hours after carbon dioxide concentration in a gas phase | | 7.23 | 25.1 2 | 0.05 | 5.63 | 1.31 | 0.26 | 1.75 | 0.55 | 9.09 |
| Intercept 0.5 hours after carbon dioxide concentration in a gas phase, ppmv | | 936. 73 | 697. 95 | 942. 05 | 103 6.5 | 103 1.68 | 996. 32 | 994.1 1 | 1001. 58 | 907.6 8 |
| $PCO_2(0)^*$, ppmv | | 937 | 698 | 942 | 103 7 | 103 2 | 996 | 994 | 1002 | 908 |
| $PCO_2(60)^*$, ppmv | | 137 1 | 220 5 | 945 | 137 4 | 111 0 | 101 2 | 1099 | 1035 | 1453 |
| $PCO_2(60)-PCO_2(0)$, ppmv | | 434 | 150 7 | 3 | 338 | 79 | 16 | 105 | 33 | 545 |
| Calcium carbonate deposition speed calculated from $PCO_2$, mg/L CaCO₃/hour | | **6.6** | **19.6** | **0.1** | **4.8** | **1.3** | **0.3** | **1.8** | **0.6** | **8.5** |
| * Total dissolved solids (mg/L) as 0.7 * Conductivity (μs/cm) | | | | | | | | | | |

FIG. 5 lists a schematic view of a comparison of calcium carbonate deposition rates calculated according to the present invention and by conventional alkalinity titration.

As can be seen from this test,

1) In terms of $CaCO_3$, the absolute error of alkalinity titration is around 5 mg/L, i.e. the resolution of the calcium carbonate deposition rate calculated by the alkalinity method is less than 5 mg/L/hour.

2) As can be seen from the two repeating data of the 25 bbp, 75 bbp, 100 ppb inventive method, the resolution of the inventive method is around 2 mg/L/hour.

3) With the adding amount of 0 ppb ATMP, the deviation of the results obtained by the two methods was relatively large, about 5 mg/L/hour. The probable reason is that since the sample with no scale inhibitor remained at a higher deposition rate after 1 hour, the sample filtration took about 15 minutes and each alkalinity measurement took about 5 minutes, so the data measured by the alkalinity method is actually about 1.5 hours, 25/1.5 = 17mg/L/hour, which is close to that measured by the method of the present invention.

4) The present invention does not require a high-intensity alkalinity titration, has a small amount of work, has continuous test data, does not require manual intervention, and has high reliability.

**Example 3**

Specific examples of the application of the present invention in Process 2 (in-situ application for testing the crystallization reaction characteristics of inorganic salts) are further described below.

In a certain circulating cooling water, municipal tap water was used as make-up water, and phosphorus-free circulating water was used for corrosion and scale inhibition treatment. It was found that the scaling control of some high-temperature heat exchangers was not good, and the treatment scheme of scale inhibition and corrosion inhibitor was proposed to be adjusted. The water quality analysis results of field water are shown in Table 5. According to the water quality and water supply source, the main scaling inorganic salt of the circulating water was calcium carbonate.

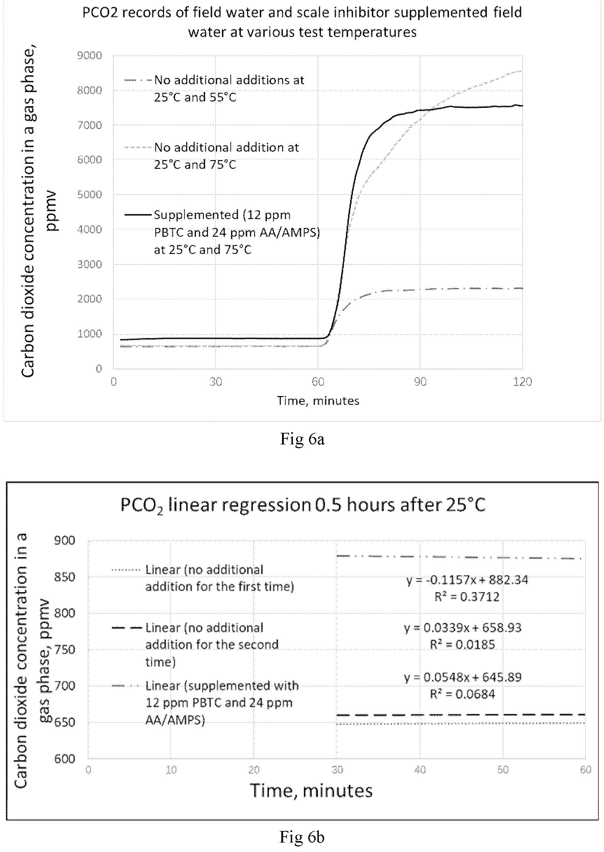

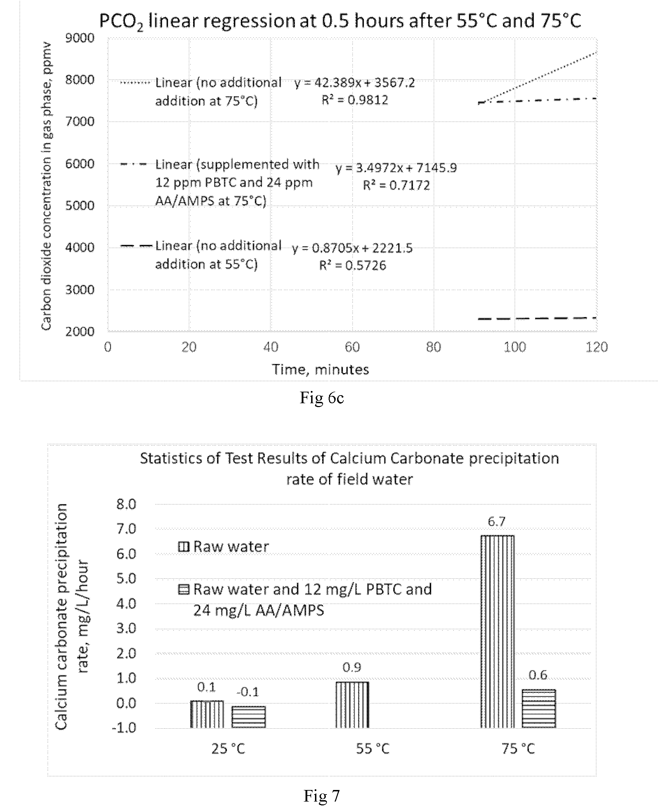

Take the field circulating water, use the method of the present invention to measure the field water and the field water supplemented with scale inhibitor at 25°C, 55°C and 75°C, according to the method of the present invention, calculate with the precipitation rate calculation method for inorganic salt precipitated type 1 calcium carbonate. Table 5 shows the experimental setup and results. FIG. 6a is a $PCO_2$ recording of field water and scale inhibitor supplemented field water at various test temperatures. FIG. 6b is the $PCO_2$ linear regression for the last 0.5 hours measured at 25°C, and FIG. 6c is the $PCO_2$ linear regression for the last 0.5 hours measured at 55°C and 75°C. FIG. 7 is a statistical plot of the results of calcium carbonate precipitation rate calculations for Type 1 field water. It can be seen from FIG. 7 that at 25°C and 55°C, the field water precipitation rate as calcium carbonate is less than 0.9 mg/L/hour. However, as the temperature increases to 75°C, the field water precipitation rate as calcium carbonate without supplementing scale inhibitor increases rapidly to 6.7 mg/L/hour, with the scale inhibitor supplemented still remaining at about 0.6 mg/L/hour, which is comparable to that without scale inhibitor at 55°C. For high-temperature heat exchangers, the current on-site chemical treatment scheme needs to be adjusted. One method is to increase the concentration of scale inhibitor. Other methods can also be used in view of the cost of the reagent. For example, the pH is lowered. On-site After adjusting the treatment protocol, water may again be taken to determine the precipitation speed of inorganic salts using the method of the present invention until an optimal protocol for inorganic salt precipitation control is found.

Table 5 Field Water Test Arrangement and Results

| Test No. | 1# | 2# | 3# |
|---|---|---|---|
| Abbreviation | No additional additions of 25°C and 55°C | No additional addition at 25°C and 75°C | Add 12 ppmPBTC and 24 ppm AA/AMPS 25°C and 75°C |
| Test time | 60 minutes + 60 minutes | | |
| Initial Cmalk, mg/L as $CaCO_3$ | 280 | | |
| $Ca^{2+}$, mg/L as $CaCO_3$ | 490 | | |
| Total dissolved solids *, mg/L | 1427 | | |
| Conductivity, $\mu$s/cm (room temperature) | 2039 | | |
| Test pH Measured@Room Temperature | 8.68 @24°C | | |
| SI of $CaCO_3$ @25 C by aqion | 1.82 | | |

(continued)

| Test No. | 1# | 2# | 3# |
|---|---|---|---|
| Abbreviation | No additional additions of 25°C and 55°C | No additional addition at 25°C and 75°C | Add 12 ppmPBTC and 24 ppm AA/AMPS 25°C and 75°C |
| supplemented PBTC, mg/L | 0 | 0 | 12 |
| Supplemented with AA/AMPS, mg/L | 0 | 0 | 24 |
| Test temperature, °C | 25 | | |
| Slope half hour after $PCO_2$, ppmv/hour | 0.0548 | 0.0339 | -0.1157 |
| Intercept Half Hour After $PCO_2$, ppmv | 646 | 659 | 882 |
| $PCO_2(0)$, ppmv | 646 | 659 | 882 |
| $PCO_2(60)$, ppmv | 649 | 661 | 875 |
| $PCO_2(60)-PCO_2(0)$, ppmv | 3 | 2 | -7 |
| Calcium Carbonate precipitation speed, mg/L $CaCO_3$/hour | **0.1** | **0.1** | **-0.1** |
| Test temperature, °C | 55 | 75 | |
| Slope half hour after $PCO_2$, ppmv/hour | 0.8705 | 42.389 | 3.4972 |
| Intercept Half Hour After $PCO_2$, ppmv | 2222 | 3567 | 7146 |
| $PCO_2(60)$, ppmv | 2274 | 6111 | 7356 |
| $PCO_2(120)$, ppmv | 2378 | 8654 | 7566 |
| $PCO_2(60)-PCO_2(120)$, ppmv | 104 | 2543 | 210 |
| Calcium Carbonate precipitation speed, mg/L $CaCO_3$/hour | **0.9** | **6.7** | **0.6** |
| *Total dissolved solids as conductivity * 0.70 | | | |

From the above experiments, it can be seen that in order to test the above data, the present invention takes only 6 hours, the calculation process is fully programmed, and only the initial Cmalk (or pH, or DIC) and TDS (which can be converted by conductivity) of water are manually input. Frequent, complex alkalinity or calcium ion analysis is not required.

Although the present invention has been described with reference to preferred embodiments, it is not to be restricted by the embodiments but only by those skilled in the art. Accordingly, the scope of the present invention should be determined by that of the appended claims.

**Claims**

1. A measurement method for the precipitation speed of an inorganic salt in water, **characterized by** comprising the following steps:

   S1: obtaining the type of an inorganic salt precipitated in water to be measured, one of inorganic carbon methyl orange alkalinity (Cmalk), a pH value, and total dissolved inorganic carbon (DIC) in the water, total dissolved solid (TDS) an element of a target inorganic salt contributing to non-inorganic carbon methyl orange alkalinity and an initial content of the element;
   S2: continuously measuring a carbon dioxide concentration ($CO_2$w) in the water to be measured or a change value of a carbon dioxide concentration ($PCO_2$) in a gas phase in balance it over time;
   S3: obtaining the precipitation amount of the target inorganic salt according to data obtained in step S1 and step S2; and
   S4: obtaining the precipitation speed of the target inorganic salt according to the precipitation amount obtained in step S3 and the time in S2.

2. The measurement method according to claim 1, **characterized in that** the other two of the inorganic carbon methyl orange alkalinity (Cmalk), the $pH(t_0)$ value and the total dissolved inorganic carbon (DIC) in the water at $t_0$ according to the following formulas in step S1,

$$DIC = CO_2w(1 + k'_1/(H^+) + k'_1k'_2/(H^+)^2), \qquad ----(1)$$

$$Cmalk = 50000CO_2w(k'_1/(H^+) + 2k'_1k'_2/(H^+)^2), \qquad ----(2)$$

$$pH = -\log(H^+) \qquad ----(3)$$

where DIC is the total dissolved inorganic carbon concentration in the water (mol/L), $CO_2w$ is the carbon dioxide concentration in the water to be measured (mol/L), Cmalk is the inorganic carbon methyl orange alkalinity in the water (mg/L, calculated as calcium carbonate), $H^+$ is a hydrogen ion concentration in the water to be measured (mol/L), $k'_1$ is a first dissociation constant of carbonic acid, and $k'_2$ is a second dissociation constant of carbonic acid.

3. The measurement method according to claim 2, **characterized in that** the target inorganic salt is calcium carbonate, and there are no other ions contributing to the inorganic carbon methyl orange alkalinity in the water to be measured, and the step S3 comprises the following steps:

S311: knowing $Cmalk(t_0)$ and $CO_2w(t_0)$ at $t_0$, and calculating $H^+(t_0)$ and $DIC(t_0)$ at the starting time $t_0$ according to formulas (2) and (1);

S312: knowing $CO_2w(t_1)$ at $t_1$, calculating $H^+(t_1)$ at $t_1$ according to formula (5), calculating $Cmalk(t_1)$ at $t_1$ according to formula (2), and calculating $DIC(t_1)$ at $t_1$ according to formula (4),

$$(DIC(t_0)-DIC(t_1)) \times 100 \times 1000 = (Cmalk(t_0)-Cmalk(t_1)) \qquad --(4)$$

$$2 \times CO_2w(t_0) - 2 \times CO_2w(t_1) = CO_2w(t_1) \times k'1/H^+(t_1) - CO_2w(t_0) \times k'1/H^+(t_0) --(5)$$

S313: calculating the calcium carbonate precipitation amount according to formula (6)

$$CaCO_3(s)(mg/L) = (DIC(t_0)-DIC(t_1)) * 10^5 \qquad --(6)$$

4. The measurement method according to claim 2, **characterized in that** the inorganic salt precipitated is calcium phosphate, and no ions are contributing to the inorganic carbon methyl orange alkalinity other than the inorganic carbon methyl orange alkalinity contributed by orthophosphate in the water to be measured, and the step S3 comprises the following steps:

S321: knowing $Cmalk(t_0)$ and $CO_2w(t_0)$ at $t_0$, and calculating $H^+(t_0)$ and $DIC(t_0)$ at the starting time $t_0$ according to formulas (2) and (1);

S322: knowing $CO_2w(t_1)$ at $t_1$, calculating $DIC(t_1)$ and $H^+(t_1)$ at $t_1$ according to formula (7), calculating $Cmalk(t_1)$ at $t_1$ according to formula (2),

$$DIC(t_0) = DIC(t_1) = CO_2w(t_1) \times (1 + k'1/H^+(t_1) + k'1k'2/(H^+(t_1)^2) --(7)$$

S323: knowing $Tp(t_0)$ at $t_0$, obtaining $DIP(t_0)$ according to formula (9), calculating $Pmalk(t_0)$ according to formulas (10)-(12), calculating $DIP(t_1)$ according to formulas (8)-(12), and calculating calcium phosphate precipitation amount according to formula (14);

$$(DIP(t_0)-DIP(t_1))/2 \times 2 \times 50 \times 1000 = Cmalk(t_0) + Pmalk(t_0) - Cmalk(t_1) - Pmalk(t_1) --(8)$$

$$DIP = Tp/95/1000 \qquad ------------------------------------------------(9)$$

$$Pmalk = (PO_4^{3-}) \times 2 \times 50000 + (HPO_4^{2-}) \times 1 \times 50000----------------(10)$$

$(PO_4{}^{3-})= Tp/95/1000 \times kp1 \times kp2 \times kp3/((H+)^{\wedge}3 + (H+)^{\wedge}2 \times kp1 + (H+) \times kp1 \times kp2 + kp1 \times kp2 \times kp3)$ 

(11)

$$(HPO_4{}^{2-})= (PO_4{}^{3-}) \times (H^+)/kp_3 \text{-------------------------------------------}(12)$$

Calcium phosphate precipitation amount $Ca_3(PO_4)_2(s)(mg/L)=(DIP(t_0)-DIP(t_1))/2 \times 310 \times 1000$

----(14)

wherein Pmalk is the inorganic phosphorus methyl orange alkalinity (mg/L, calculated as calcium carbonate), DIP is the total dissolved inorganic phosphorus concentration (mol/L), and Tp is the total dissolved inorganic phosphorus concentration (mg/L, calculated as $PO_4{}^{3-}$); $kp_1$ is the first ionization constant of phosphoric acid, $kp_2$ is the second ionization constant of phosphoric acid, and $kp_3$ is the third ionization constant of phosphoric acid;.

5. The measurement method according to claim 2, **characterized in that** the target inorganic salt is magnesium silicate, and no ions are contributing to the inorganic carbon methyl orange alkalinity other than the inorganic carbon methyl orange alkalinity contributed by silicate in the water to be measured, and the step S3 comprises the following steps:

S331: knowing $Cmalk(t_0)$ and $CO_2(t_0)$ at $t_0$, and calculating $H^+(t_0)$ and $DIC(t_0)$ at the starting time $t_0$ according to formulas (2) and (1);
S332: knowing $CO_2(t_1)$ at $t_1$, calculating $DIC(t_1)$ and $H^+(t_1)$ at $t_1$ according to formula (7), calculating $Cmalk(t_1)$ at $t_1$ according to formula (2),

$$DIC(t_0) = DIC(t_1) = CO_2(t_1) \times (1+k'1/H^+(t_1)+k'1k'2/(H^+(t_1)^{\wedge}2)) \text{ --}(7)$$

S333: knowing $Tsi(t_0)$ at $t_0$, obtaining $DISi(t_0)$ according to formula (16), calculating $Simalk(t_0)$ according to formulas (17) and (18), and calculating $DISi(t_1)$ according to formulas (15)-(18), and calculating the magnesium silicate precipitation amount according to formula (19);

$$(DISi(t_0) - DISi(t_1)) \times 2 \times 50 \times 1000 = Cmalk(t_0) + Simalk(t_0) - Cmalk(t_1) - Simalk(t_1) \text{ --}(15)$$

$$DISi = Tsi/60/1000 \qquad \text{-- (16)}$$

$$Simalk = 50000 \times (Si(OH)_3O^-) \qquad \text{--}(17)$$

$$(Si(OH)_3O^-)= DISi/(1 + H^+/Ksi) \qquad \text{--}(18)$$

magnesium silicate precipitation amount $MgSiO_{3\ (s)}(mg/L)= (DISi(t_0)-DISi(t_1) \times 2 \times 50 \times 1000$

-- (19)

wherein DISi is the total dissolved inorganic silicon content (mol/L), Tsi is the total dissolved inorganic silicon content (mg/L, calculated as $SiO_2$), Simalk is the inorganic silicon methyl orange alkalinity (mg/L, calculated as calcium carbonate), and Ksi is the dissociation constant of silicic acid; .

6. The measurement method according to claim 2, **characterized in that** the target inorganic salt is colloidal silicon, and no ions are contributing to the inorganic carbon methyl orange alkalinity other than the inorganic carbon methyl orange alkalinity contributed by silicate in the water to be measured, and the step S3 comprises the following steps:

S341: knowing $Cmalk(t_0)$ and $CO_2w(t_0)$ at $t_0$, and calculating $H^+(t_0)$ and $DIC(t_0)$ at the starting time $t_0$ according to formulas (2) and (1);
S342: knowing $CO_2(t_1)$ and $Cmalk(t_1)$ at $t_1$, calculating $DIC(t_1)$ and $H^+(t_1)$ at $t_1$ according to formula (7), calculating

Cmalk($t_1$) at $t_1$ according to formula (2);

$$DIC(t_0) = DIC(t_1) = CO_2(t_1) \times (1+k'1/H^+(t_1)+k'1k'2/(H^+(t_1)^\wedge 2) \quad ----(7)$$

S343: knowing Tsi($t_0$) at $t_0$, obtaining DISi($t_0$) according to formula (16), calculating Simalk($t_0$) according to formulas (17) and (18), and calculating DISi($t_1$) according to formulas (16)-(18) and (20), and calculating the colloidal silicon precipitation amount according to formula (21);

$$(DIsi(t_0) - DIsi(t_1)) \times 1 \times 50 \times 1000 = -(Cmalk(t_0) + Simalk(t_0)) +(Cmalk(t_1)-Simalk(t_1)) \quad --(20)$$

$$DISi = Tsi/60/1000 \qquad --(16)$$

$$Simalk = 50000 \times (Si(OH)_3O^-) \quad --(17)$$

$$(Si(OH)_3O^-)= DISi/(1 + H^+/Ksi) \qquad --(18)$$

colloidal silicon precipitation amount $SiO_2(s)(mg/L)= (DISi(t_0) - DISi(t_1)) \times 1 \times 60 \times 1000$ --(21)

7. The measurement method according to claim 1, **characterized in that** the step S2 comprises: measuring a carbon dioxide gas concentration $PCO_2$ in a closed detector, wherein a gas circulation pump and a carbon dioxide concentration in a gas phase detector are provided in a loop of the closed detector, and continuously recording the carbon dioxide gas concentration $PCO_2$ during measurement; then converting the carbon dioxide gas concentration $PCO_2$ into the carbon dioxide concentration $CO_2w$ in the water according to Henry's law.

8. An electronic device, **characterized by** comprising: a processor; a memory, and a computer program stored in the memory and operable on the processor, when the computer program is executed by the processor, the measurement method for the precipitation speed of an inorganic salt in water according to any one of claims 1 to 7 is implemented.

9. A computer-readable storage medium, **characterized in that** a computer program is stored in the storage medium, and when the computer program is executed by a processor, the measurement method for the precipitation speed of an inorganic salt in water according to any one of claims 1 to 7 is implemented.

10. A measurement system for the precipitation speed of an inorganic salt in water, **characterized by** comprising a closed detector for measuring the carbon dioxide concentration and the electronic device according to claim 8, the electronic device further comprising an input-output module, the output end of the closed detector being in signal communication with the input-output module of the electronic device.

11. The measurement system according to claim 10, **characterized in that** the closed detector comprises a test container, a carbon dioxide detector, a first constant-temperature heating unit, and a gas circulation pump, wherein water to be measured is placed in the test container, an inlet of the carbon dioxide detector is in communication with the test container, an outlet of the carbon dioxide detector is in communication with the test container via the gas circulation pump to form a pipeline loop for recirculating carbon dioxide, and the first constant-temperature heating unit is provided on the test container to control the temperature of the measured water in a constant temperature state.

S1

Obtain the type of an inorganic salt precipitated in water to be measured, one of inorganic carbon methyl orange alkalinity (Cmalk), a pH value, and total dissolved inorganic carbon (DIC) in said water, a total dissolved solid (TDS) content, an element of a target inorganic salt contributing to non-inorganic carbon methyl orange alkalinity and the content of the element;

S2

Continuously measure a change value of a carbon dioxide concentration (CO2w) in said water or a carbon dioxide concentration (PCO2) in a gas phase in balance with said water over time in a closed constant-temperature and constant-pressure environment;

S3

Obtain the precipitation amount of the target inorganic salt according to data obtained in step S1 and step S2;

S4

Obtain the precipitation speed of the target inorganic salt according to the precipitation amount obtained in step S3 and the time in S2

Fig 1

Fig 2

Fig 3

Fig 4a

Fig 4b

Fig 5

Fig 6a

Fig 6b

**PCO$_2$ linear regression at 0.5 hours after 55°C and 75°C**

········· Linear (no additional addition at 75°C)    $y = 42.389x + 3567.2$    $R^2 = 0.9812$

— · — Linear (supplemented with 12 ppm PBTC and 24 ppm AA/AMPS at 75°C)    $y = 3.4972x + 7145.9$    $R^2 = 0.7172$

— — Linear (no additional addition at 55°C)    $y = 0.8705x + 2221.5$    $R^2 = 0.5726$

Fig 6c

**Statistics of Test Results of Calcium Carbonate precipitation rate of field water**

Raw water

Raw water and 12 mg/L PBTC and 24 mg/L AA/AMPS

Fig 7

## INTERNATIONAL SEARCH REPORT

| International application No. |
| --- |
| **PCT/CN2023/135108** |

### A. CLASSIFICATION OF SUBJECT MATTER

G01N33/18(2006.01)i

According to International Patent Classification (IPC) or to both national classification and IPC

### B. FIELDS SEARCHED

Minimum documentation searched (classification system followed by classification symbols)

IPC:G01N

Documentation searched other than minimum documentation to the extent that such documents are included in the fields searched

Electronic data base consulted during the international search (name of data base and, where practicable, search terms used)

CNABS; CNTXT; CNKI; 读秀, DUXIU; 万方, WANFANG: 结垢, 沉积, 二氧化碳, 碱度; VEN; WOTXT; USTXT; WEB OF SCIENCE: scale, precipitation, CO2, alkalinity

### C. DOCUMENTS CONSIDERED TO BE RELEVANT

| Category* | Citation of document, with indication, where appropriate, of the relevant passages | Relevant to claim No. |
| --- | --- | --- |
| PX | CN 115856245 A (HE GAORONG et al.) 28 March 2023 (2023-03-28)<br>claims 1-11 | 1-11 |
| Y | US 4277343 A (PAZ JACOB D.) 07 July 1981 (1981-07-07)<br>description, column 2, line 48-column 3, line 8, and column 7, lines 33-47 | 1-11 |
| Y | WOJTOWICZ, John A. "Calcium Carbonate Precipitation Potential"<br>*Journal of the Swimming Pool and Spa Industry,*<br>Vol. 2, No. 2, 31 December 2001 (2001-12-31), pages 51-57<br>page 51, abstract, pages 51-52, Carbonate Equilibria part, and page 53, last paragraph | 1-11 |
| A | GB 202206475 D0 (UNIVERSITÄT HAMBURG et al.) 15 June 2022 (2022-06-15)<br>entire document | 1-11 |
| A | US 2020364623 A1 (SAUDI ARABIAN OIL COMPANY) 19 November 2020 (2020-11-19)<br>entire document | 1-11 |
| A | WO 2014155868 A1 (MITSUBISHI ELECTRIC CORP.) 02 October 2014 (2014-10-02)<br>entire document | 1-11 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 February 2024** | **22 February 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/135108**

| Patent document cited in search report | | | Publication date (day/month/year) | Patent family member(s) | | | Publication date (day/month/year) |
|---|---|---|---|---|---|---|---|
| CN | 115856245 | A | 28 March 2023 | None | | | |
| US | 4277343 | A | 07 July 1981 | None | | | |
| GB | 202206475 | D0 | 15 June 2022 | US | 2023357037 | A1 | 09 November 2023 |
| | | | | EP | 4276070 | A1 | 15 November 2023 |
| US | 2020364623 | A1 | 19 November 2020 | US | 11867027 | B2 | 09 January 2024 |
| | | | | WO | 2020232089 | A1 | 19 November 2020 |
| WO | 2014155868 | A1 | 02 October 2014 | EP | 2980584 | A1 | 03 February 2016 |
| | | | | EP | 2980584 | A4 | 02 November 2016 |
| | | | | EP | 2980584 | B1 | 12 September 2018 |
| | | | | JP | 6029744 | B2 | 24 November 2016 |
| | | | | JPWO | 2014155868 | A1 | 16 February 2017 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB T166322008 A

**Non-patent literature cited in the description**

- **V. TANTAYAKOM**. Scale inhibition study by turbidity measurement. *Journal of Colloid and Interface Science*, 2005, vol. 284, 57-65